(19) Europäisches Patentamt / European Patent Office / Office européen des brevets

(11) EP 1 357 944 B1

# (12) EUROPEAN PATENT SPECIFICATION

(45) Date of publication and mention of the grant of the patent:
**13.06.2007 Bulletin 2007/24**

(21) Application number: **02701466.1**

(22) Date of filing: **29.01.2002**

(51) Int Cl.:
***A61K 41/00*** *(2006.01)* ***A61P 37/00*** *(2006.01)*

(86) International application number:
**PCT/IB2002/000267**

(87) International publication number:
**WO 2002/060482 (08.08.2002 Gazette 2002/32)**

(54) **PERYLENEQUINONES FOR USE WITH IMMUNOTHERAPY AGENTS**

PERYLENEQUINONE ZUR ANWENDUNG MIT IMMUNOTHERAPEUTISCHEN MITTELN

PERYLENEQUINONES DESTINEES A ETRE UTILISEES COMMENT AGENTS IMMUNOTHERAPEUTIQUES

(84) Designated Contracting States:
**AT BE CH CY DE DK ES FI FR GB GR IE IT LI LU MC NL PT SE TR**

(30) Priority: **30.01.2001 US 264677 P**

(43) Date of publication of application:
**05.11.2003 Bulletin 2003/45**

(73) Proprietor: **Altachem Pharma Ltd.**
**Alberta T5S 1G2 (CA)**

(72) Inventor: **LEVEUGLE, Beatrice**
**Edmonton, Alberta T5S 1G2 (CA)**

(74) Representative: **Alcock, David**
**D Young & Co**
**120 Holborn**
**London, EC1N 2DY (GB)**

(56) References cited:

| | |
|---|---|
| **WO-A-01/12217** | **WO-A-98/33470** |
| **WO-A-98/52609** | **WO-A-99/65517** |
| **WO-A-02/060483** | **WO-A-02/062386** |

- **MILLER GERALD G ET AL: "Immunophotodynamic therapy: Current developments and future prospects." DRUG DEVELOPMENT RESEARCH, vol. 42, no. 3-4, November 1997 (1997-11), pages 182-197, XP001051166 ISSN: 0272-4391**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Technical Field of the Invention

The invention involves compositions and methods for treating diseases and the like by administering compounds that are both photosensitizers and sonosensitizers, and potentiating any immunotherapies used to treat said disease (s). The compositions and methods are particularly suited to treating cancers in humans and animals, and to modulating the function of the immunotherapeutic agent.

### Background of the Invention

Immunotherapy is based on the principle of inducing or activating the immune system to recognize and eliminate undesirable cells, such as neoplastic cells. The key elements in any immunotherapy is to induce or trigger the host immune system to first recognize a molecule as an unwanted target, and then to induce the system to initiate a response against that molecule. In healthy hosts, the immune system recognizes surface features of a molecule that are not a normal constituent of the host (i.e., is "foreign" to the host). Once the recognition function occurs, the host must then direct a response against that particular foreign molecule.

Both the recognition and the response elements of the immune system involve a highly complex cascade of biological reactions. In most immunologically based disorders, at least one of the steps in the recognition phase, or at least one of the steps in the response phase, are disrupted. Virtually any disruption in either of these complex pathways leads to a reduced response or to the lack of any response. The inability of the immune system to destroy a growing tumor has been attributed, among other factors, to the presence of tumor-associated antigens (TAA) that induce immunological tolerance and/or immunosuppression. For example, in some kinds of cancer, the cancer itself misleads the host into accepting the foreign cancer cell as a normal constituent, thus disrupting the recognition phase of the immune system. The immunological approach to cancer therapy involves modification of the host-tumor relationship so that the immune system is induced or amplifies its response to the TAAs. If successful, inducing or amplifying the immune system can lead to tumor regression, tumor rejection, and potentially, to tumor cure.

The ability to up- or down-regulate immune responses and to control potentially auto-reactive immunocompetent cells is vital for normal immune function and survival. Regulatory mechanisms include the induction of clonal anergy (via inappropriate antigen-presenting cells), peripheral clonal deletion/apoptosis, cytokine (e.g. transforming growth factor-beta (TGF-β) or IL-10)-induced non-responsiveness, 'veto' cells, auto-reactive cytolytic T cells, and both non-specific and antigen-specific T suppressor cells. At least in theory, each of these regulatory systems provides a mechanistic basis for therapeutic intervention.

The ideal cancer treatment modality should not only cause tumor regression and eradication but also induce a systemic anti-tumor immunity, which is essential for control of metastatic tumors and for long term tumor resistance. In this regard, photodynamic therapy (PDT) and sonodynamic therapy (SDT) represent a promising new approach(es) for the treatment of cancer. These therapies involve systemic or topical administration of a sensitizer, followed by its activation by light of a specific wavelength (PDT), or activation by sound of a specific frequency (SDT). The activation of the sensitizer leads to the production of activated oxygen and radical species that initiate a cascade of biochemical reactions, resulting in direct cell destruction, damage to the tumor vasculature and immune inflammatory responses. The induction of an inflammatory response and the generation of tumor specific immunity were suggested to play a decisive role in achieving long-term tumor control [1, 2]. This concept is supported by preclinical and clinical studies. For instance it has been shown that the therapeutic efficacy of PDT is greatly attenuated in immuno-compromised mice (nude and SCID) as compared with wild-type mice, and the adoptive transfer of T-cells or bone marrow cells into immuno-compromised mice were effective in delaying the recurrence of PDT-treated tumors [2, 3]. In addition, Abdel-Hady et al (2001) have recently demonstrated that in patients with vulval intraepithelial neoplasia, the clinical response to ALA-based PDT can be correlated with the number of infiltrating immune cells and HLA class I expression [4].

The inflammatory reaction is believed to represent a critical initial development that orchestrates events leading to the recognition of antigens of PDT-treated tumors and the ensuing generation of a long lasting tumor immune response [5-9]. PDT-induced photo-oxidative damage results in the release of a plethora of pro-inflammatory mediators liberated from cancer cell membranes, vascular endothelium and tumor stromal elements and the subsequent invasion of the tumor site by neutrophils and other myeloid effector cells. A number of cytokines are produced within the tumor after PDT treatment. The activated immune cells together with the liberation of cytokines instigate and amplify the acute inflammatory reaction into the targeted lesion. PDT-released tumor cell debris, cytokines and infiltrating immune cells capable of engulfing and presenting tumor antigens to T-lymphocytes, might create a unique environment for promoting cell-mediated immunity and the induction of a long lasting immune response

The activity of neutrophils, macrophages and CTLs was found to contribute to the therapeutic outcome of PDT [3, 6, 10-12]. Neutrophils and macrophages accumulate in the tumor area as early as 5 min after PDT treatment. These

cells may kill tumor cells directly through their direct cytolytic activity or indirectly through cooperation with lymphoid cells and participate in the development of cancer-specific immunity [2, 5]. The depletion of neutrophils in tumor bearing mice as well as the blocking of cell adhesion molecules engaged in the recruitment of these leukocytes in tissues was found to decrease PDT mediated anti-tumor effects [8, 13]. Similarly, the inactivation of macrophages by silica treatment also reduces the cures of PDT-treated tumors [14]. Using techniques of bone marrow transplantation and adoptive splenocyte/Tcell transfer between immunocompetent and immunodeficient mice, as well as specific depletion of CD4+ and CD8+ cells, it has been demonstrated that lymphoid cell activity is required for the PDT-mediated tumor cure [12, Korbelik, 1996 #15, 14]. Moreover, it has also been observed that PDT generates tumor-specific T-lymphocytes that can be recovered from distant lymphoid sites, such as lymph nodes or spleen, even after protracted times after light treatment [2, 3].

The photosensitizing and therapeutic properties of natural perylenequinonoid pigments (PQPs), such as hypocrellins, in biological systems have been recognized during the past two decades. See Diwu, et al., J. Photochem. Photobiol. A: Chem., 64:273 (1992); Zhang et al., (1989); and Wan, et al., "Hypocrellin A, a new drug for photochemotherapy," Kexue Tongbao (English edition) 26:1040 (1981). For their general chemical properties [see Weiss, et al., Prog. Chem. Org. Nat. Prod., 52:1 (1987) and Diwu, et al., Photochem & Photobiol., 52:609-616 (1990)]. PQP's general photophysical and photochemical properties have been reviewed in Diwu, et al., Pharmac. Ther., 63:1 (1994). Hypocrellins belong to the general class of perylenequinonoid pigments, and include hypocrellin A (HA) and hypocrellin B (HB).

PQPs are of interest because they may be administered in an un-activated (or non-toxic) state, and then subsequently activated. PQPs, and hypocrellin derivatives in particular, are also of interest because they can be activated using different modalities, for example, using light, sound, or combinations thereof.

Sonodynamic activation of sensitizers has been found to be useful since ultrasound has the appropriate tissue attenuation coefficient for penetrating intervening tissues to reach desired treatment volumes, while retaining the ability to focus energy on reasonably small volumes. Diagnostic ultrasound is a well accepted, non-invasive procedure widely used in the developed world, and is considered safe even for fetal imaging. The frequency range of diagnostic ultrasound lies between 100 kHz -12 MHz, while 50 kHz sound provides enough energy to effect cellular destruction through microregional cavitation.

The biological effects of exposure to ultrasound are the result of its physical and chemical effects. The most obvious biological effects of ultrasound treatment stem from heating of the medium through which it passes. Such heating is exploited during physiotherapy to help heal injured tissues (Lehmann et al., 1967; Patrick, 1966), and has been investigated as a possible modality for tumor treatment. This is due to the sensitivity of many tumors to hyperthermia, a state in which tissue temperatures are elevated above 42°C (Doss and McCabe, 1976; Marmor et al., 1979; Sculier and Klastersky, 1981; Bleehen, 1982; Hynynen and Lulu, 1990). Ultrasound has also been used in combination with radiation therapy to improve treatment response *in vivo* compared to radiotherapy alone (Clarke et al., 1970; Repacholi et al., 1971; Mitsumori et al., 1996). A principal danger in the use of ultrasound for therapeutic purposes is the formation of 'hotspots' due to regions of constructive interference and preferential absorption of ultrasonic energy by bone regions with low curvature radii (Lehmann et al., 1967; Linke et al., 1973). These hotspots can cause serious damage to nearby tissues (Hill, 1968; Bruno et al., 1998).

Summary of the Invention

In accordance with the present invention, there is provided the <Claim 1>. Such derivatives of perylenequinone pigments (PQPs) having both photosensitizing properties and sonosensitizing properties are used to treat diseases and other conditions by modulating the body's existing immune system, and/or any immunotherapies used to treat the disease or other condition.

In another aspect, the present invention provides <Claim 12>.

The inflammatory/immune character of PDT makes it particularly suitable for being combined with various forms of immunotherapy that can effectively improve the cure rates of treated tumors. A variety of immunotherapy treatments were shown to be effective in conjunction with PDT. This include adoptive transfer of immune cells [3, 15], the use of different cytokines [16,17], and a variety of vaccines serving as non-specific enhancers of immune response. With respect with the latter, the beneficial effect on PDT-mediated tumor response was reported for adjuvant treatments with the Bacillus Calmette-Guerin (BCG) vaccine [18, 19], mycobacterial cell wall extract [20], and *Corynebacterium parvum* vaccine [21].

BCG is an attenuated strain of *Mycobacterium bovis* developed for the use as a vaccine against human tuberculosis. BCG is known to stimulate cell-mediated immunity, humoral immunity, and macrophage function, which can theoretically lead to increased tumor destruction. Superficial bladder cancer is one of the few human malignancies in which nonspecific immunotherapy with BCG has proven to be effective.

Hypocrellins have been selected as potential photosensitizers for PDT [22] and preclinical studies have demonstrated their potential as anti-cancer agents [23]. The present invention comprises the potentiation of the anti-tumor activity of amino-substituted hypocrellins, such as demethoxy hypocrellin B (DMHB), when used in combination with an immunotherapeutic agent, e.g., BCG.

The present invention concerns altering immunogenicity in a manner that produces a beneficial or therapeutically desirable effect. As used herein, a beneficial or desirable immune response is one that produces a therapeutically desirable result, e.g., control of tumor growth in animals or humans. A beneficial therapeutic response will typically include activation of the immune system and/or one or more of its components, induction of the immune system and/or one or more of its components, and/or a T cell immune response, and/or a humoral immune response. For example, for a cancer such as ovarian cancer, a beneficial or desirable immune response includes the production of an antibody that immunoreacts with a previously non-immunoreactive ovarian cancer antigen. In this example, the immune response to an antigen is increased. In another example, for a condition such as inflammation, a beneficial or desirable immune response includes the production of an antibody that immunoreacts with a previously immunoreactive antigen so that it becomes non-immunoreactive. In this example, the immune response is decreased. In transplantation, the immune system attacks MHC-disparate donor tissue leading to graft rejection, in autoimmune disease it attacks normal tissues, and in allergy the immune system is hyper-responsive to otherwise harmless environmental antigens. It is now recognized that immunosuppressive therapy may be appropriate for treating each of these disorders. A beneficial result may also be achieved by modulating, i.e., increasing or decreasing the activity or function of an immunotherapeutic agent itself.

The compositions of the present invention, activated by light and/or sound, exhibit substantial absorption in the red spectral region or therapeutic frequencies of ultrasound; produce high singlet oxygen yield; can be produced in pure, monomeric form; may be derivatized to optimize properties of red light absorption, ultrasound activation, tissue biodistribution, and toxicity; have reduced residual cutaneous photosensitivity; and are rapidly excreted. They afford nuclear targeting by covalent attachment to DNA minor-groove binding agents, such as stapled lexotropins, to enhance phototoxicity. They are not genotoxic. This trait is important in the context of treatment -related secondary malignancies.

The photosensitizing and sonosensitizing compounds, when administered systemically, distribute throughout the body. Over a short period, ranging from hours to days, the compounds clear from normal tissues, but are selectively retained by rapidly proliferating cells (e.g., cancer cells or psoriasis lesions) for up to several days. The PQPs are inactive and non-toxic until activated, e.g., exposed to light in a specific wavelength range, to sound in a specific frequency range, or combinations thereof.

The use of compounds that can be activated using two different activation protocols may be therapeutically beneficial. Light, which can penetrate to a surface depth of about 5 mm to about 7 mm, can activate compounds for treating surface lesions or those target cells within a certain distance of a light source. Ultrasound, on the other hand, can penetrate deep within the body to treat deeply seated cells, such as tumor masses inaccessible to a source of light.

The compounds are also beneficial therapeutically due to their dual selectivity. The compounds are selective in their ability to preferentially localize the drug at the site of a predetermined target, such as a cancer cell, and they are selective in that precise delivery of light and/or sound can be confined to a specific area.

Brief Description of the Drawings

Figure 1 shows the structures for naturally occurring hypocrellin (Fig. 1A), and exemplary synthetic derivatives, HBBA-R2 (Fig. 1B), HBEA-R1 (Fig.1C), and HBDP-R1 (Fig. 1 D).

Figure 2 shows several structures for the demethoxylated HB compounds of the present invention, where $R_1$, $R_2$, $R_3$, $R_4$ are $OCH_3$ or $NHCH_2Ar$ (Ar are phenyl or pyridyl group), $NHCH(CH_2)_n$ (where $-CH(CH_2)_n$ are alicyclic group and n=3,4,5,6). 2-BA-2-DMHB is where $R_1$, $R_2$, $R_3$ are $OCH_3$, and $R_4$ is $NH(CH_2)_3CH_3$. Alternatively, $R_1$, $R_2$, $R_3$, $R_4$ may be $OCH_3$ or $NHCH_2(CH_2)_nAr$, wherein Ar is a phenyl, naphthyl, polycyclic aromatic or a heterocyclic moiety, and n is 0-12.

Figure 3 shows the percent of increase in tumor volume for PDT alone and PDT in combination with BCG, for animals responding only partially to PDT treatment. Figure 3A shows the results as an X-Y plot, and Figure 3B shows the results as a bar graph.

Modes For Carrying Out the Invention

The present invention comprises the use of amino-substituted hypocrellins, perylenequinone (PQP) derivatives, as photodynamic and sonodynamic agents, and the therapeutic use of the derivatives according to the invention as immune system modulators. The preferred compounds for use in the present invention are amino substituted hypocrellins derivatives selected from the group consisting of HBBA-R2, HBEA-R1, HBDP-R1, and DMHB.

A composition may be used for treating a pre-determined disease or condition comprising administering a composition comprising a perylenequinone derivative, allowing the perylenequinone derivative to distribute to a portion of the body, preferably throughout the body, and activating the perylenequinone derivative.

In the present invention, the perylenequinone derivatives are amino-substituted hypocrellins. In the most preferred embodiments of the invention, the perylenequinone derivatives are demethoxylated hypocrellins (see Figure 2), where $R_1$, $R_2$, $R_3$, $R_4$ are $OCH_3$ or $NHCH_2Ar$ (Ar are phenyl or pyridyl group), $NHCH(CH_2)_n$ (where $-CH(CH_2)_n$ are alicyclic group and n=3,4,5,6). 2-BA-2-DMHB is where $R_1$, $R_2$, $R_3$ are $OCH_3$, and $R_4$ is $NH(CH_2)_3CH_3$. Alternatively, $R_1$, $R_2$, $R_3$, $R_4$ may be $OCH_3$ or $NHCH_2(CH_2)_nAr$, wherein Ar is a phenyl, naphthyl, polycyclic aromatic or a heterocyclic moiety, and n is 0-12.

The present invention also includes compositions for altering the immunogenic state of the host organism. In altering the immunogenic state, the compositions increase, decrease, or maintain the host's immunogenic state, and/or increase, decrease, or maintain the function of the immunotherapeutic agent. An example of deriving a therapeutic benefit by increasing the immunogenicity includes but is not limited to treatments for cancer or some infectious diseases, such as hepatitis. An example of decreasing the immunogenicity includes but is not limited to treatments for rheumatoid arthritis, An example of maintaining immunogenicity includes but is not limited to supplemental treatments for patients that have become tolerant to antigens after an initial response. In a most preferred embodiment of the invention, the methods and compositions do not decrease the antigenicity of the active component in the therapeutic composition.

The compositions may also be used in combination with other administered immunotherapies. For example, the compositions may be used with antibody, antigen, cytokine, and/or immunoadjuvant based immunotherapies. The compositions modulate the function or activity of the immunotherapy itself. Exemplary immunotherapies or immunomodulators are described in Mandell, Principles and Practice of Infectious Diseases, $5^{th}$ edition. Exemplary immunomodulators include, but are not limited to, BCG, Granulocyte colony-stimulating factor (filgrastim), Granulocyte-macrophage colony-stimulating factor (sargramostim), Interferon alfa, Interferon alfa-2a, Interferon alfa-2b, Interferon alfacon-1, Interferon alfa-n3, Intravenous immunoglobulin, and imiquimod.

The present invention may also increase the over-all host response to a disease or condition, and produce a therapeutic benefit for the recipient.

The present invention also includes compositions that result in the induction of a beneficial immune response, particularly where one skilled in the art would not expect to find an antigen-specific immune response, e.g., tumor-associated antigens ("self") antigens.

The PQP may be conjugated to a targeting agent, such as an antibody, antibody receptor, or the like, or a fragment thereof, the use of the conjugate for potentiating the immune system, and activating the conjugate using light and/or sound.

Potentiating the immune system, as used herein, refers to modifying the host-tumor relationship by modulating (inducing, amplifying, and/or inactivating) the response of the immune system to cancer associated antigens. Such potentiating of the immune system leads to tumor regression, rejection, and possibly cure. Potentiating the immune system also refers to modulating the activity of various immune system components, including but not limited to antibodies, antigens, cytokines, immunoadjuvants, and the like. It is believed that potentiating the immune system leads to macrophage accumulation at the tumor site as well as distant metastases.

The present invention concerns potentiating the immune system in a manner that produces a beneficial or therapeutically desirable effect. As used herein, a beneficial or desirable immune response is one that produces a therapeutically desirable result. A beneficial therapeutic response will typically include modulation of the immune system and/or one or more of its components, e.g., activating or inactivating an existing immune response. Modulation may include induction of the immune system and/or one or more of its components, and/or a T cell immune response, and/or a humoral immune response. The immune response to an antigen may be increased or decreased, depending on which response provides a beneficial result.

As used herein, a comprehensive approach to providing a therapeutic benefit involves one or more, or all, of the following: cellular immunity and the molecules involved in its production; humoral immunity and the molecules involved in its production; ADCC immunity and the molecules involved in its production; CDC immunity and the molecules involved in its production; natural killer cells; and cytokines and chemokines, and the molecules and cells involved in their production. One skilled in the art will recognize that a beneficial immune response (and thereby overcoming immunotolerance) may be determined by a number of means. Activation of the multiple arms of the immune systems may be determined, for example, by measuring the pre- and post-treatment antigen specific immune response. Specific demonstrations of the induction of a beneficial immune response would include one or more of the following:

1) a humoral response to the administered immunogen including evidence of antibody;
2) a humoral response to the antigen, including evidence of the appearance of antigen-specific antibodies to the same and/or different epitopes on the antigen as the epitope for the binding agent;
3) antibody-dependent cytotoxicity, including evidence that post-injection serum with an antigen-specific antibody titer mediates tumor killing when the serum is incubated with peripheral blood mononuclear cells and tumor cell targets relative to pre-injection baseline serum;
4) complement-dependent cytotoxicity, including evidence that post injection serum combined with complement-containing plasma kills tumor cell targets relative to pre-injection baseline serum;

5) natural killer cell activity, including enhanced tumor cell killing by peripheral blood mononuclear cells (containing NK cells) in post-injection blood samples taken prior to the appearance of a measurable antibody response to the tumor associated antigen (TAA) relative to pre-treatment peripheral blood mononuclear cells;
6) antigen-enhanced cytotoxicity, including enhanced tumor cell target killing by peripheral blood mononuclear cells (in the presence of TAA-positive tumor cells) relative to pre-administration levels; and
7) cellular immunity, including evidence of T cell proliferation or tumor cell lysis post-injection relative to pre-injection.

As used herein, "perylenequinone derivative" or "derivative" refers to all compounds derived from native or natural perylenequinones (PQPs) and which can be activated by light of a pre-determined wavelength and/or by sound of a pre-determined frequency. The derivative may be a compound derived from naturally occurring quinone compounds. A derivative may also be complexed with or include other active reagents, including but not limited to chemotherapeutic agents or alkylating agents. PQPs include, but are not limited to hypocrellins, cercosporin, phleichrome, cladochrome, elsinochromes, erythroaphins, and calphostins.

As used herein, "perylenequinone derivative" or "derivative" also refers to all compounds derived from native or natural perylenequinones and which can be activated by light of a pre-determined wavelength and/or sound of a pre-determined frequency. The derivative may be a compound derived from naturally occurring hypocrellin A or hypocrellin B, and hypocrellin-like compounds. A derivative may also be complexed with or include other active reagents, including but not limited to chemotherapeutic agents or alkylating agents. As noted in more detail below, the composition containing a PQP active agent may include a wide variety of additional components, including, for example, one or more of gases, gaseous precursors, liquids, oils, stabilizing materials, diagnostic agents, photoactive agents, bioactive agents and/or targeting ligands.

In a preferred embodiment of the invention, the PQP derivative is an amino acid derivative of hypocrellin B. At the present time, the most preferred immunoconjugates use hypocrellin B functionalized to have an acid, acid bromide, hydrazine, thiol, or primary amine antibody binding site.

A hypocrellin derivative used in the present invention also includes 2-butylamino-2-demethoxy-hypocrellin B (2-BA-2-DMHB). 2-BA-2-DMHB exhibits strong absorption in the red spectral region. Compared with its parent compound HB, its absorption bands extend toward longer wavelengths. The extinction coefficient at 583 nm was 2.5-fold as much as HB at 548 nm, and at 621 nm was over 3.8-fold as much as HB at 580 nm. This characteristics means that DMHB will exhibit more favorable tissue penetration, and therefore may be greater clinical significance.

The compounds used in the present invention may be produced by any method that results in a purified or substantially purified compound, or in a compound that is useful as a photodynamic or sonodynamic agent. The compounds may also form a composition comprising a cocktail of compounds, i.e., more than one compound. These methods are well known in the art, e.g., Liu, et al., "Synthetic studies in novel hypocrellin B derivatives," Tetrahedron, 49:10785 (1993); and Diwu, et al., Anti-Cancer Drug Design, 8:129-143 (1993). Hypocrellin derivatives may be readily synthesized from the parent compound, hypocrellin B (HB), a natural product of the fungus *Hypocrella bambuase sacc.,* a phytopathogen of bamboo. HB derivatives, HBBA-R2 (butylaminated HB), HBDP-RI (2-(N,N-dimethylamino)-propylamine-HB), and HBEA-RI (ethanolaminated HB) were prepared by amination of the phenolic hydroxyl groups of the parent compound.

Many of POP's properties are summarized in Diwu, et al., J. Photochem. Photobiol. A: Chem., 64:273 (1992). Some perylenequinones are also potent inhibitors of certain viruses, particularly human immunodeficiency virus (HIV), and also the enzyme protein kinase C (PKC). Both anti-HIV and anti-PKC activities of certain POPs are light dependent, a phenomenon implicated in the photodynamic therapy of cancers [Diwu, et al., Biochem. Pharmacol., 47:373-389 (1994)]. The Diwu et al paper also discloses the successful conjugation of HB to a protein.

The PQP derivatives may be functionalized, e.g., include reactive groups including but not limited to an acid, hydroxyl, an acid halide (preferably bromide), a hydrazine, a thiol, or a primary amine. The binding reagent may include reactive groups including but not limited to amino acids, such as cysteine, lysine, aspartic acid, glutamic acid and other dicarboxylic acid amino acids, and other tri- or poly-functional amino acid derivatives.

The perylenequinone derivatives used in the present invention are particularly suited for therapeutic use because they exhibit absorption and phototoxic activity in the phototherapeutic window (about 560 nm to about 700 nm); exhibit excellent sonodynamic activity in a frequency range from about 1 MHz to about 3 MHz; are low molecular weight, typically from about 550 daltons to about 880 daltons); are available in pure monomeric form; exhibit rapid serum and skin clearance; have negligible cytotoxicity *in vitro* and *in vivo;* have excellent photopotentiation (e.g., two orders of magnitude), so the safety margin in use is excellent; phototoxicity is mediated through conventional type II reactions and Type I reactions (indicating utility for hypoxic tumor cells); are potent inhibitors of protein kinases; confer apoptotic cell death *in vitro* and *in vivo*; exhibit no genotoxicity; exhibit excellent tumor control; may be molecularly configured for targeted delivery: may be targeted to nuclear regions to further augment sono/phototoxicity; and the parent hypocrellins are amenable to site-specific modification, so that many derivatives may be formed, derivatives with varying degrees of photosensitizing and/or sonosensitizing characteristics.

The composition containing a PQP active agent may include a wide variety of additional components, including,

for example, one or more of gases, gaseous precursors, liquids, oils, stabilizing materials, diagnostic agents, photoactive agents, bioactive agents and/or targeting ligands.

As used herein, "disease" refers to the management, diagnosis, and/or palliation of any mammalian (including human) disease, disorder, malady, or condition that can be treated by photodynamic and/or sonodynamic therapy. "Disease" includes but is not limited to cancer and its metastases, such as skin cancer; growths or tumors, and their metastases; tumors and tumor cells, such as sarcomas and carcinomas, including solid tumors, blood-bome tumors, and tumors found in nasal passages, the bladder, the esophagus, or lung, including the bronchi ; viruses, including retroviruses; bacterial diseases; fungal diseases; and dermatological conditions or disorders, such as lesions of the vulva, keloid, vitiligo, psoriasis, benign tumors, endometriosis, Barett's esophagus, *Tinea capitis,* and lichen amyloidosis.

As used herein, "administering" refers to any action that results in exposing or contacting one or more PQP derivatives with a pre-determined cell, cells, or tissue, typically mammalian. As used herein, administering may be conducted *in vivo, in vitro,* or *ex vivo.* For example, a composition may be administered by injection or through an endoscope. Administering also includes the direct application to cells of a composition. For example, during the course of surgery, tumor cells may be exposed. These exposed cells (or tumors) may be exposed directly to a composition of the present invention, e.g., by washing or irrigating the surgical site and/or the cells.

As used herein, activation, activating, or similar terms refers to the use of light waves and/or sound frequency to make a compound or portion of a compound more chemically reactive. Any method for applying a light source and/or a sound source to a perylenequinone derivative may be used, e.g., direct application, an ultrasound machine, focused ultrasound, high-intensity focused ultrasound, and illuminating endoscopy, to name a few.

Upon application of the appropriate light or sound, the sensitizers can chemically (e.g., through oxidation, reduction and the like) change into a form that is toxic, and/or modulates an immune response. For example, following excitation of a photosensitizer or a sonosensitizer to a long-lived excited triplet state, a targeted tumor is destroyed either by the highly reactive singlet oxygen species (a Type II mechanism) and/or by free radical products (a Type I mechanism) generated by quantum energy transfer. Major biological target molecules of the singlet oxygen species and/or free radical products include nucleic acids, enzymes and cell membranes. A secondary therapeutic effect of such methods involves the release of pathophysiologic products, such as prostaglandins, thromboxanes and leukotrienes, by tissue exposed to the effects of activated photosensitizers.

Activating a sensitizer using light and activating a sensitizer using sound may be used together since each of the individual procedures are complementary. That is, red, visible light suitable for activating a perylenequinone derivative is capable of penetrating into tissue or into a body from about 5 mm to about 7 mm, and sound suitable for activating a perylenequinone derivative is capable of fully penetrating into tissue or into a body.

As used herein, "photopotentiation factor" refers to the property of the compound(s) to exert light- or sound-mediated toxicity in excess of its (their) inherent unactivated toxicity. The activation factor may be calculated as the ratio of the $LD_{50}$ of cells treated without activation to the $LD_{50}$ of the cells treated with an activated compound (drug $LD_{50}$ divided by activated drug $LD_{50}$). Where the term "$LD_{50}$" has been used above, the term "$IC_{50}$" may be substituted, to address the bioassays that concern metabolic activity rather than the endpoint of lethality, loss of reproductive capability, or clonogenic death. The relative photoactivation efficiency of a compound may also be determined using a clonogenic assay, an assay that is well known to those skilled in the art.

A desirable PQP derivative is one that is non-toxic (or of low toxicity) at high drug concentrations without activation, i.e., without light (also referred to as "dark"), and/or without sound, and is toxic at low concentrations when light of the appropriate wavelength, or sound of the appropriate frequency, is applied. As is recognized by those skilled in the art, the most desirable compounds are those that provide a wide range of non-toxic doses in an un-activated state, as this characteristic provides an increased safety factor for the patient.

As used herein, physiologically acceptable fluid refers to any fluid or additive suitable for combination with a composition containing a PQP derivative. Typically these fluids are used as a diluent or carrier. Exemplary physiologically acceptable fluids include but are not limited to preservative solutions, saline solution, an isotonic (about 0.9%) saline solution, or about a 5% albumin solution or suspension. It is intended that the present invention is not to be limited by the type of physiologically acceptable fluid used. The composition may also include pharmaceutically acceptable carriers. Pharmaceutically accepted carriers include but are not limited to saline, sterile water, phosphate buffered saline, and the like. Other buffering agents, dispersing agents, and inert non-toxic substances suitable for delivery to a patient may be included in the compositions of the present invention. The compositions may be solutions, suspensions or any appropriate formulation suitable for administration, and are typically sterile and free of undesirable particulate matter. The compositions may be sterilized by conventional sterilization techniques.

The sensitizer may be administered to the patient by any biologically suitable route. For example, the sensitizer may be introduced into the patient by intravenous, subcutaneous, intraperitoneal, intrathecal, intravesical, intradermal, intramuscular, or intralymphatic routes. The composition may be in solution, tablet, aerosol, or multi-phase formulation forms. Liposomes, long-circulating liposomes, immunoliposomes, biodegradable microspheres, micelles, or the like may also be used as a carrier, vehicle, or delivery system. Furthermore, using *ex vivo* procedures well known in the art, blood

or serum from the patient may be removed from the patient; optionally, it may be desirable to purify the antigen in the patient's blood; the blood or serum may then be mixed with a composition that includes a sensitizer according to the invention; and the treated blood or serum is returned to the patient. The clinician may compare the anti-idiotypic and anti-isotypic responses associated with these different routes in determining the most effective route of administration. The invention should not be limited to any particular method of introducing the sensitizer into the patient.

Intracellular uptake may be rapid (e.g., within about 2 hours), or uptake may require more time (e.g., about 20 hours or more). Some degree of selective tumor uptake might be achieved by modification of the pKa of the sensitizer, since the interstitial milieu of some tumors is more acidic than that of normal tissues. This invention includes a method for identifying compounds where the toxicity of the compounds is higher for cancer cells than for normal cells, via comparative clonogenic assays.

Adjuvants or immunoadjuvants are defined as a group of structurally heterogeneous compounds, used to evoke or increase an immune response to an antigen. Theoretically, each molecule or substance that is able to favor or amplify a particular situation in the cascade of immunological events, ultimately leading to a better immunological response, can be defined as an adjuvant [schijns 2000]. Classically recognized examples include oil emulsions, saponins, aluminium or calcium salts, non-ionic block polymer surfactants, derivatives of lipopolysaccharides (LPS), mycobacteria and many others. Adjuvants may potentiate the immune response by enhancing antigen localization (aluminum compounds, liposomes, water-and-oil emulsion [Freund's incomplete adjuvant]); enhancing antigen presentation (interferon gamma interferon inducers, beryllium, muramyl dipeptide, Freund's complete adjuvant); and activating lymphocytes (interleukins-1 and -2). [Lise LD, Audibert F. Immunoadjuvants and analogs of immunomodulatory bacterial structures. Curr Opin Immunol 1989;2:269-274]

The PQP derivatives may also be used in conjunction with and conjugated to a number of other compounds, signaling agents, enhancers, and/or targeting agents. For example, a hypocrellin derivative used in the present invention may be conjugated to an antibody, preferably a monoclonal antibody, or a compound such as transferrin. The binding agent includes any DNA minor-groove targeting agent, such as lexotropsin or netropsin, preferably to enhance the toxicity through the cell nucleus. Suitable enhancers include but are not limited to pKa modifiers, hypoxic cell radiosensitizers, and bioreductively activated anti-neoplastic agents, such as mitomycin C (preferably to effect or potentiate the toxicity of the compound in hypoxic cells or microorganisms). Suitable signaling agents include but are not limited to markers of apoptotic cell death or necrotic cell death, or regulatory molecules endogenous to cell cycle control or delay, preferably to potentiate the phototoxicity or sonotoxicity of the compound(s) by induction of apoptotic or necrotic cell death, or by inhibition of the repair of any form of lethal or potentially lethal damage (PLD).

As noted above, an embodiment of the invention includes binding agent-PQP conjugates (or immunoconjugates) and the therapeutic use of these conjugates. In accordance with the present invention, any method of linking a binding agent to a PQP may be used. For example, it is well known how to link an antibody or an antibody fragment to a photosensitizer. For example, Goff, et al., British Journal of Cancer, 74:1194-1198 (1996) discloses the production of an immunoconjugate by incubating a photosensitizer with monoclonal antibody OC125, an antibody that specifically binds to the CA125 antigen associated with most ovarian cancers. In this exemplary immunoconjugate, polyglutamic acid may be bound to a monoethylendiamine monoamide derivative, which is then covalently linked to the carbohydrate moiety at the hinge region of the monoclonal antibody away from the antigen binding sites. Other exemplary linkages are disclosed in U.S. Patent 4,722,906 and 3,959,078, both incorporated herein by reference. Briefly, these patents disclose providing a photosensitizer with a selector group, or a latent reactive group, that is the other member of a specific binding pair, e.g., a reactive group that covalently bonds to an antibody.

As is recognized by one skilled in the art, an effective dose of the derivative or a conjugate that includes the derivative will depend in part on the severity of the disease and the status of the patient's immune system. One skilled in the art will recognize that a variety of doses may be used, and are dependent on a variety of well known factors. Generally, the composition will include about 0.1 $\mu$g to about 2 mg or more of binding agent per kilogram of body weight, more commonly dosages of about 200 $\mu$g per kilogram of body weight. The concentration usually will be at least about 0.5%. Any amount may be selected primarily based on fluid volume, viscosity, antigenicity, etc., in accordance with the chosen mode of administration.

Administration of the conjugate or the derivative may be more than once, preferably three times over a prolonged period. As the compositions of this invention may be used for patients in a serious disease state, i.e., life-threatening or potentially life-threatening, excesses of the binding agent may be administered if desirable. Actual methods and protocols for administering pharmaceutical compositions, including dilution techniques for injections of the present compositions, are well known or will be apparent to one skilled in the art. Some of these methods and protocols are described in Remington's Pharmaceutical Science, Mack Publishing Co. (1982).

A composition of the present invention may be administered alone, or in combination (sequentially or in batch) with other immunotherapeutic compositions. These features afford potential augmentation of the photodynamic and/or sonodynamic therapeutic ratio through sequential sensitizer administration (followed by light treatment). Under these conditions, a distant metastasis may be targeted.

This can comprises administering a first active agent, preferably having a slow uptake, and administering a second active agent, preferably having a more rapid uptake than that of the first agent. Both first and second active agents may then be activated by exposing the patient and/or the agent to light of suitable wavelength, and/or to sound of a suitable frequency, as described above.

Buffers are used primarily to stabilize a formulation against the chemical degradation that might occur if the pH changed appreciably. Buffer systems employed normally have as low a buffer capacity as feasible in order to not disturb significantly the body buffer systems when injected. The buffer range and effect of the buffer on activity must be evaluated. Appropriate adjustment is useful to provide the optimum conditions for pH dependent partition into the target malignant tissues or lesion area. Examples of such buffer systems include the following acids: acetic, adipic, ascorbic, benzoic, citric, glycine, lactic, tartaric, hydrochloric, phosphoric, sulfuric, carbonic and bicarbonic; and their corresponding salts such as: potassium, sodium, magnesium, calcium and diethanolamine salts.

Examples

Example 1. Direct amination of hypocrellin B.

HB (50 mg) was dissolved in ethanol (5 mL) containing the amine (1 mL), and the resulting solution was refluxed for 6-18 h depending upon the individual amine used. The mixture was poured into ice-water, neutralized with 10% hydrochloric acid, and extracted with chloroform. The chloroform layer was washed with water and dried with anhydrous $Na_2SO_4$ and evaporated to afford a blue solid. The solid was first chromatographed on a 1% $KH_2PO_4$-silica gel column with dichloromethane-methanol (gradient ratio) as an eluent to give several constituents. Each constituent was twice rechromatographed on 1% citric acid-silica gel plate using 6:1:1 petroleum ether-ethyl acetate-ethanol as developing agent to afford the individual derivatives.

Example 2. Amination of hypocrellin B with ethanolamine.

Reaction of HB with ethanolamine according to the above procedure affords five products. HBEA-R2 and HBEA-R1 (Diwu et al. 1993) were identified and characterized as follows:

HBEA-R2 (20%): R: 3270, 1717 and 1612 $cm^{-1}$ ; $^1H$-NMR (in DMSO-$d_6$): 11.46 (s, <1H, exchangeable with $D_2O$, phenolic OH), 1.38 (s, <1H, exchangeable with $D_2O$, phenolic OH), 6.83 (s, 1H, ArH), 6.78 (s, 1 H, ArH), 4.09 (s, 3H, $OCH_3$),3H, $OCH_3$), 3.94 (s, 3H, $OCH_3$), 3.92 (s, 3H, $OCH_3$), 3.85- 3.50 (m, 4H, $2NHCH_3$), 3.40-2.92 (m, 4H, $CH_2OH$), 2.11 (s, 3H, $COCH_3$) and 1.72 ppm (s, 3H, $CH_3$). MS (FAB): 615 (M+H). Calculated for $C_{34}H_{34}$ $N_2O_9$: 614.2264; found, 614.2270.

HBEA-R1 (Isomer B)] (12%): IR: 3260, 1720 and 1613 $cm^{-1}$; $^1H$-NMR (in OMSO-$d_6$): 12.11 (s, <1H, exchangeable with $D_2O$, phenolic OH), 11.99 (s, <1H, exchangeable with $D_2O$, phenolic OH), 6.47 (s, 1H, ArH), 6.35 (s, 1 H, ArH), 4.03 (s, 3H, $OCH_3$, 3.95 (s, 6H, 2 x $OCH_3$), 3.93 (s, 3H, $OCH_3$), 3.88-3.62 (m, 4H, $2NHCH_3$), 3.20-2.95 (m, $2CH_2OH$, 2.15 (s, 3H, $COCH_3$) and 1.90 ppm (s, 3H, $CH_3$). MS (FAB): 615 (M+H). Calculated for $C_{34}H_{34}N_2O_9$: 614.2264; found; 614.2268.

Example 3. Amination of hypocrellin B with butylamine.

Synthesis of HBBA-R2 (Isomer A) and 3-Acetyl-4,6,8,9,11,13-hexamethoxy-2-methyl-1 H-cyclohepta[ghi]perylene-5,12-dione (Diwu et al. 1993). Reaction of HB with butylamine according to i.e. above procedure afforded five products. Two of these compounds were identified as follows:

HBBA-R2 (21%): IR: 3280, 1702 and 1616 $cm^{-1-}$; $^1H$-NMR: 15.65 (s, 1H, exchangeable with $D_2O$, phenolic OH), 14.94 (s, 1 H, exchangeable with $D_2O$, phenolic OH), 6.41 (s, 1 H, ArH), 6.40 (s, 1 H, ArH), 4.07 (s, 3H, $OCH_3$), 4.00 (s, 3H, $OCH_3$), 3.96 (s, 3H, $OCH_3$), 3.93 (d, 3H, $OCH_3$), 3.24 (m, 4H, $2NHCH_2$), 1.98 (s, 3H, $COCH_3$). 1.26 (s, 3H, $CH_3$) and 1.70- 0.85 ppm (m, 14H, $2CH_2CH_2CH_3$). MS (FAB): 639 (M+H). Calculated for $C_{38}H_{42}N_2O_7$: 638.2992; found; 638.2998.

HBBA-R1 (11%): IR: 3300, 1715 and 1616 $cm^{-1}$, $^1H$-NMR: 15.40 (s, 1 H, exchangeable with $D_2O$, phenolic OH), 15.18 (s, 1H, exchangeable with $D_2O$, phenolic OH), 6.48 (s, 1H, ArH, 6.33 (s, 1 H, ArH), 4.01 (s, 6H, 2 x $OCH_3$), 3.97 (d, 1 H, CH), 3.96 (s, 6H), 2 x $OCH_3$), 3.54 (m, 4H, $2NHCH_2$), 3.14 (d, 1 H, CH), 2.16 (s, 3H, $COCH_3$), 1.69 (s, 3H, $CH_3$) and 1.60-0.85 ppm (m, 14H, $2CH_2CH_2CH_2$). MS (FAB): 639 (M+H). Calculated for $C_{38}H_{42}N_2O_7$: 639.2998; found; 638.2992.

Example 4.

***Tumor model.*** Mammary sarcoma EMT6 tumor cells were passaged in syngeneic BALB/c mice and the tumor cells isolated from the dissected tumor were kept frozen in liquid nitrogen. For the experiment cells were thawed and cultured in Waymouth's medium until subconfluent. A suspension of $10^5$ tumor cells in PBS was inoculated s.c. into the mouse flank. Tumors were treated 8 days after inoculation, when the tumor volume reached a size of ~ 70 $mm^3$. Mice were divided into 2 groups of 5 mice each.

***PDT treatment.*** In this experiment, all mice (10) received PDT treatment. The skin overlying the tumor was shaved and a fixed dose of DMHB freshly resuspended in mineral oil was administrated i.p (50 μM total body, 200 μL/ mouse). After 24 h the mice were anesthetized with methophane, and the tumor was subjected to 635 nm light delivered by optical fiber from the Biolitec laser. The power at the illuminated spot (2 cm) was 150 mW. A dose of 100 Joules was given for each tumor.

***BCG treatment****.* BCG treatment was given to only one group of mice (PDT-BCG group). Bacillus Calmette-Guérin (BCG) vaccine (OncoTICE, Organon, Canada Ltd.) was used as a single subtumoral administration by lifting the subcutaneous tumor and slowly injecting $10^7$ cfu in sterile injectable saline (50 μL volume) below the lesion. The BCG injection was performed immediately after PDT treatment.

***Tumor response evaluation****.* Tumor response to therapy was evaluated by monitoring the mice for signs of tumor growth every second day. Changes in tumor volumes were determined by measuring with a caliper the lesion's three orthogonal diameters. The tumor volume was calculated from the expression

$$V = \pi/6 \times d_1 \times d_2 \times d_3$$

Where V = volume ($mm^3$) and $d_{1-3}$ are the three orthogonal diameters (mm).

**RESULTS**

In the present example determined whether the combination of hypocrellin DMHB with BCG could improve the therapeutic potential of DMHB. The photosensitizer dose of 50 μM and the conditions for light treatment were chosen based on previous *in vivo* studies performed with the DMHB derivative HBEA-R1 [23]. The potentiation of PDT activity by BCG has been previously described for other photosensitizers [19] and the same BCG treatment was adapted to our protocol.

Mice bearing EMT6 tumor were randomly divided into two groups of 5 mice each. The first group of mice (PDT group) received DMHB alone whereas the second group (PDT-BCG) received DMHB in combination with BCG. The activation of DMHB by light treatment was identical in the two groups. The day before the mice were exposed to light (day 0, no therapeutic effect could be observed at this point since neither the DMHB was activated or BCG injected), we observed that the mean tumor volume of the PDT-BCG group was higher (52.73 ± 8 $mm^3$) compared to the PDT group (30.37 ± 4 $mm^3$). In order to express the results in the most accurate way, the resultas are expressed as % of tumor increase, rather than directly as tumor volume. Indeed larger tumors will grow much faster compared to smaller ones and a beneficial therapeutic effect in the PDT-BCG group, if not dramatically significant, therefore can easily be masked by this effect. The % of tumor increase in the other hand is more representative of the pace at which the tumor grow and therefore, we believe is a more accurate way to represent the difference obtained between the two groups. The tumor volume was calculated from the expression:

$$\% \text{ increase} = 100 \times [\text{tumor volume on day of measurement} \div \text{Tumor volume on day 0}]$$

The efficacy of DMHB alone and in combination with BCG in the control of EMT6 tumor is represented for each individual mouse in Figure 3. A considerable delay in tumor growth was observed when PDT was used in combination with BCG compared to PDT alone.

In Figure 3 the values obtained represent the beneficial effect of combined PDT-BCG treatment compared to PDT alone in animals responding only partially to PDT treatment. Results represented in figure 3 A and B indicate that in animals responding only partially to PDT treatment, a decrease of approximately 50 % in tumor growth is obtained when PDT is used in combination with BCG compared to PDT alone.

This example shows that very encouraging results were obtained in this initial study with PDT therapy combined with BCG showing beneficial anti-tumor effect compared to PDT therapy.

Example 5.

Hypocrellin B (HB) was prepared by quantitative potassium hydroxide dehydration of hypocrellin A (HA) followed by neutralization with HA, chloroform extraction, and recrystallization with benzene-petroleum ether, 2-butylamino-2-demethoxy-hypocrellin B (2-BA-2-DMHB) was prepared by reflux with n-butylamine in pyridine, neutralization, and chloroform extraction of HB. The product was subjected to 1 % citric acid-silica gel thin-layer chromatography (TLC), using a 6:1:1 mixture of petroleum ether/ethyl acetate/ethanol (95%) as eluent, and three compounds were obtained. They were the target compound (rate of flow (Rr) =0.64) and two by-products (Rr= 0.74 and 0:40. respectively), which were identified by satisfactory NMR, mass spectra and elemental analysis. The target compound was further purified with TLC and the desired product, 2-BA-2-DMHB, was obtained in 54% yield. The purity or HB and 2-BA-2-DMHB was assessed by high-performance liquid chromatography and found to be higher than 95%.

Example 6.

**Perylenequinonoid Pigments (Hypocrellins) and Their Photosensitizing and Sonosensitizing Properties**

| Compound | | Photosensitizing Potential* | Sonosensitizing Potential* |
|---|---|---|---|
| **DMHBa** | Demethylated-HB | 3.0μM | 1.0mM |
| **DMHBb** | 2-butylamino-2-demethoxy-Hypocrellin B | 0.1μM | 0.1mM |
| **HA** | Hypocrellin A | 4.0μM | None |
| **HBAC-R1** | Cystamine-HB isomer 1 | 1.0μM | None |
| **HBAC-R2** | Cystamine-HB isomer 1 | 5.0μM | None |
| **HBAM-R1** | 2-morpholino-ethylaminated HB | 4.0μM | None |
| **HBDD-R1** | 2-(N,N-dimethyl-amino) propylamine-Hypocrellin B | | 1.0mM |
| **HBEA-R1** | Ethanolamine-Hypocrellin B isomer 1 | 0.15μM | 1.0mM |
| **HBEA-R2** | Ethanolamine-Hypocrellin B isomer 2 | 7.50μM | None |
| **HBED-R2** | Ethylenediamne-Hypocrellin B | 4.0μM | None |
| **HBMA-IV** | Methylamine-Hypocrellin B | 1.0μM | None |
| ***Molar Concentration which exerts $LD_{50}$ in EMT6 murine mastocytoma *in vitro,* for a fixed dose of light or ultrasound** | | | |

Cited references:

1. Hunt DW, Chan AH (2000) Expert Opinion on Investigational Drugs 9: 807
2. Korbelik M (1996) Journal of Clinical Laser Medicine & Surgery 14: 329
3. Korbelik M, Dougherty GJ (1999) Cancer Research 59: 1941
4. Abdel-Hady ES, Martin-Hirsch P, Duggan-Keen M, Stern PL, Moore JV, Corbitt G, Kitchener HC, Hampson IN (2001) Cancer Research 61: 192
5. Dougherty TJ, Gomer CJ, Henderson BW, Jori G, Kessel D, Korbelik M, Moan J, Peng Q (1998) Journal of the National Cancer Institute 90: 889
6. Korbelik M, Krosl G, Krosl J, Dougherty GJ (1996) Cancer Research 56: 5647
7. Ochsner M (1997) Journal of Photochemistry & Photobiology. B - Biology 39: 1
8. de Vree WJ, Essers MC, Koster JF, Sluiter W (1997) Cancer Research 57: 2555

9. Gollnick SO, Liu X, Owczarczak B, Musser DA, Henderson BW (1997) Cancer Research 57: 3904
10. Krosl G, Korbelik M, Dougherty GJ (1995) British Journal of Cancer 71: 549
11. Korbelik M, Krosl G (1994) Photochemistry & Photobiology 60: 497
12. Hendrzak-Henion JA, Knisely TL, Cincotta L, Cincotta E, Cincotta AH (1999) Photochemistry & Photobiology 69: 575
13. de Vree WJ, Essers MC, de Bruijn HS, Star WM, Koster JF, Sluiter W (1996) Cancer Research 56: 2908
14. Korbelik M, Cecic I (1999) Cancer Letters 137: 91
15. Korbelik M, Sun J (2001) International Journal of Cancer 93: 269
16. Bellnier DA (1991) Journal of Photochemistry & Photobiology. B - Biology 8: 203
17. Krosl G, Korbelik M, Krosl J, Dougherty GJ (1996) Cancer Research 56: 3281
18. Cho YH, Straight RC, Smith JA (1992) Journal of Urology 147: 743
19. Korbelik M, Sun J, Posakony JJ (2001) Photochemistry & Photobiology 73: 403
20. Korbelik M, Cecic I (1998) Journal of Photochemistry & Photobiology. B - Biology 44: 151
21. Myers RC, Lau BH, Kunihira DY, Torrey RR, Woolley JL, Tosk J (1989) Urology 33: 230
22. Estey EP, Brown K, Diwu Z, Liu J, Lown JW, Miller GG, Moore RB, Tulip J, McPhee MS (1996) Cancer Chemotherapy & Pharmacology 37: 343
23. Miller GG, Brown K, Ballangrud AM, Barajas O, Xiao Z, Tulip J, Lown JW, Leithoff JM, Allalunis-Turner MJ, Mehta RD, Moore RB (1997) Photochemistry & Photobiology 65: 714

While the invention has been described in some detail by way of illustration and example, it should be understood that the invention is susceptible to various modifications and alternative forms, and is not restricted to the specific embodiments set forth. It should be understood that these specific embodiments are not intended to limit the invention but, on the contrary, the intention is to cover all modifications, equivalents, and alternatives falling within the spirit and scope of the invention.

## Claims

1. Use of an amino-substituted hypocrellin for the manufacture of a medicament for modulating activity of an immunotherapeutic agent, wherein the amino-substituted hypocrellin is a sonosensitizer and/or a photosensitizer, and wherein the amino-substituted hypocrellin is capable when activated of modulating the potential of an immunotherapeutic agent.

2. Use according to claim 1 wherein the amino-substituted hypocrellin may be activated using sound of a predetermined frequency.

3. Use according to claim 1 wherein the amino-substituted hypocrellin may be activated using light of a predetermined wavelength.

4. Use according to claim 1, 2 or 3 wherein an immunotherapeutic agent is selected from the group consisting of an antibody, antigen, cytokine, or immunoadjuvant.

5. Use according to any one of claims 1 to 4 wherein the amino-substituted hypocrellin is non-toxic at high concentrations in its non-activated state and toxic at low concentrations in its activated state.

6. Use according to any one of claims 1 to 5 wherein the amino-substituted hypocrellin is selected from the group consisting of butylaminated hypocrellin B; 2-(N,N-dimethylamino)-propylamine-hypocrellin B; ethanolaminated hypocrellin B; and 1,12-Bis[2-(acetyloxy)propyl]-2,4,6,7,9,11-hexamethoxy-3,10-perylenedione.

7. Use according to any one of claims 1 to 5 wherein the amino-substituted hypocrellin is selected from the compounds of the formulae

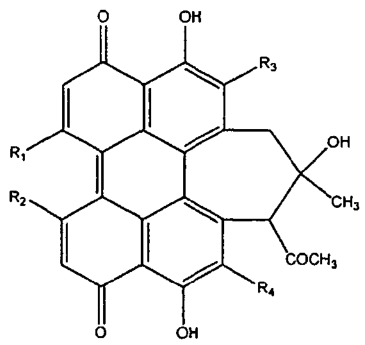

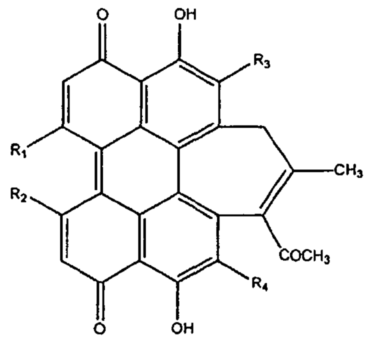

where $R_1$, $R_2$, $R_3$, $R_4$ are $OCH_3$ or $NHCH_2Ar$ (Ar are phenyl or pyridyl group), $NHCH(CH_2)_n$ (where $-CH(CH_2)_n$ are alicyclic group and n=3,4,5,6).

**8.** Use according to claim 7 wherein $R_1$ $R_2$, $R_3$ are $OCH_3$, and $R_4$ is $NH(CH_2)_3CH_3$.

**9.** Use according to any one of claims 1 to 5 wherein the amino-substituted hypocrellin is selected from the compounds of the formulae

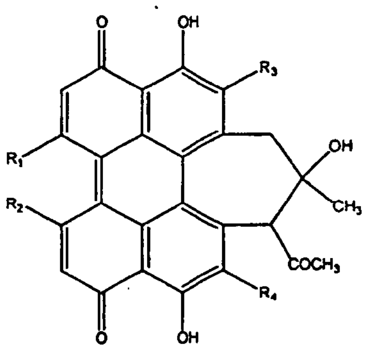

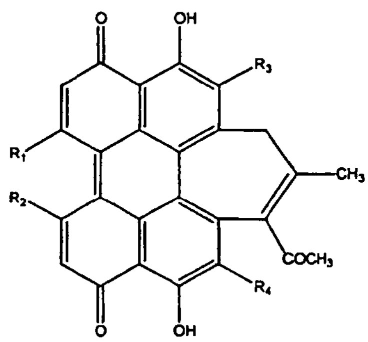

wherein $R_1$, $R_2$, $R_3$, $R_4$ are $OCH_3$ or $NHCH_2(CH_2)_nAr$, wherein Ar is a phenyl, naphthyl, polycyclic aromatic or a heterocyclic moiety, and n is 0-12.

**10.** Use of a composition comprising an amino-substituted hypocrellin and an immunotherapeutic agent for the manufacture of a medicament for modulating activity of an immunotherapeutic agent, according to any one of claims 1 to 9.

**11.** Use according to any one of claims 1 to 9 for the manufacture of a medicament for treating a condition selected from skin conditions, cancer, viral diseases, retroviral diseases, bacterial diseases, autoimmune diseases, and fungal diseases.

**12.** A product comprising

(a) an amino-substituted hypocrellin, wherein the amino-substituted hypocrellin is a sonosensitizer and/or a photosensitizer, and is capable when activated of modulating the potential of an immunotherapeutic agent, and the amino-substituted hypocrellin is selected from the compounds of the formulae

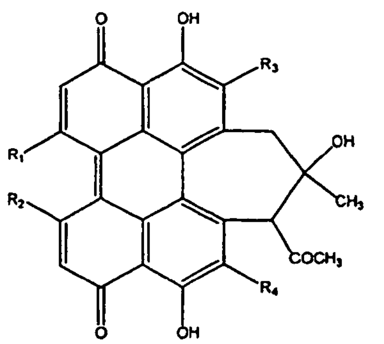

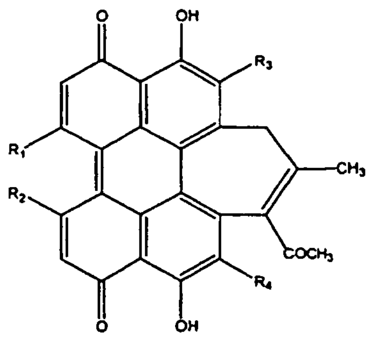

wherein

(i) $R_1$, $R_2$, $R_3$, $R_4$ are $OCH_3$ or $NHCH_2Ar$ (Ar are phenyl or pyridyl group), $NHCH(CH_2)_n$ (where $-CH(CH_2)_n$ are alicyclic group and n=3,4,5,6); or

(ii) $R_1$, $R_2$, $R_3$, $R_4$ are $OCH_3$ or $NHCH_2(CH_2)_nAr$, where Ar is a phenyl, naphthyl, polycyclic aromatic or a heterocyclic moiety, and n is 0-12;

(b) an immunotherapeutic agent,

for simultaneous or sequential use in modulating the activity of an immunotherapeutic agent or the treatment of a condition selected from skin conditions, cancer, viral diseases, retroviral diseases, bacterial diseases, autoimmune diseases, and fungal diseases.

## Patentansprüche

1. Verwendung eines aminosubstituierten Hypocrellins für die Herstellung eines Medikaments zur Modulation der Aktivität eines immuntherapeutischen Mittels, wobei das aminosubstituierte Hypocrellin ein Sonosensibilisator und/ oder ein Photosensibilisator ist und wobei das aminosubstituierte Hypocrellin, wenn es aktiviert wird, das Potential eines immuntherapeutischen Mittels modulieren kann.

2. Verwendung nach Anspruch 1, wobei das aminosubstituierte Hypocrellin unter Verwendung von Schall mit einer vorbestimmten Frequenz aktiviert werden kann.

3. Verwendung nach Anspruch 1, wobei das aminosubstituierte Hypocrellin unter Verwendung von Licht mit einer vorbestimmten Wellenlänge aktiviert werden kann.

4. Verwendung nach Anspruch 1, 2 oder 3, wobei ein immuntherapeutisches Mittel aus der Gruppe ausgewählt ist, bestehend aus einem Antikörper, einem Antigen, einem Zytokin oder einem Immunadjuvans.

5. Verwendung nach einem der Ansprüche 1 bis 4, wobei das aminosubstituierte Hypocrellin bei hohen Konzentrationen in seinem nicht-aktivierten Zustand nicht toxisch ist und bei niedrigen Konzentrationen in seinem aktivierten Zustand toxisch ist.

6. Verwendung nach einem der Ansprüche 1 bis 5, wobei das aminosubstituierte Hypocrellin aus der Gruppe ausgewählt ist, bestehend aus butylaminiertem Hypocrellin B, 2-(N,N-Dimethylamino)-propylamin-hypocrellin B, ethanolaminiertem Hypocrellin B und 1,12-Bis-[2-(acetyloxy)-propyl]-2,4,6,7,9,11-hexamethoxy-3,10-perylendion.

7. Verwendung nach einem der Ansprüche 1 bis 5, wobei das aminosubstituierte Hypocrellin aus den Verbindungen mit den folgenden Formeln ausgewählt ist

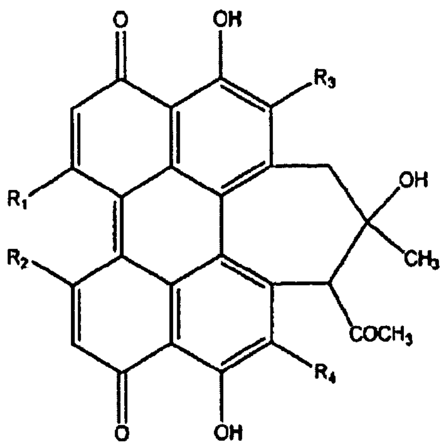

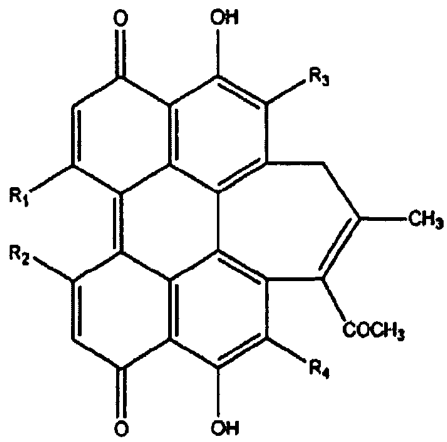

wobei $R_1$, $R_2$, $R_3$, $R_4$ $OCH_3$ oder $NHCH_2Ar$ (Ar ist eine Phenyl- oder Pyridylgruppe), $NHCH(CH_2)_n$ (wobei $-CH(CH_2)_n$ eine alizyklische Gruppe ist und n = 3, 4, 5, 6 ist) sind.

**8.** Verwendung nach Anspruch 7, wobei $R_1$, $R_2$, $R_3$ $OCH_3$ sind und $R_4$ $NH(CH_2)_3CH_3$ ist.

**9.** Verwendung nach einem der Ansprüche 1 bis 5, wobei das aminosubstituierte Hypocrellin aus den Verbindungen der folgenden Formeln ausgewählt ist

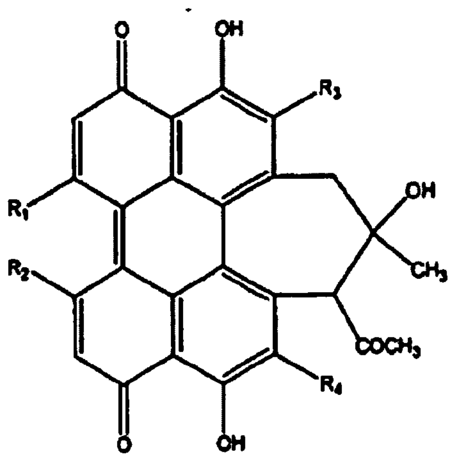

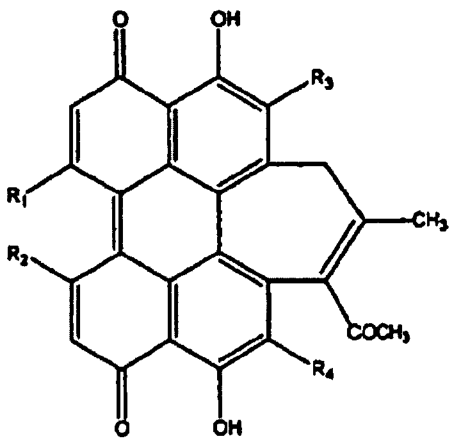

wobei $R_1$, $R_2$, $R_3$, $R_4$ $OCH_3$ oder $NHCH_2(CH_2)_nAr$ sind, wobei Ar ein Phenyl-, ein Naphthyl-, ein polyzyklischer aromatischer Rest oder ein heterozyklischer Rest ist und n 0-12 ist.

**10.** Verwendung einer Zusammensetzung, welche ein aminosubstituiertes Hypocrellin und ein immuntherapeutisches Mittel umfaßt, für die Herstellung eines Medikaments zur Modulation der Aktivität eines immuntherapeutischen Mittels, gemäß einem der Ansprüche 1 bis 9.

**11.** Verwendung nach einem der Ansprüche 1 bis 9 für die Herstellung eines Medikaments zur Behandlung eines Zustands, ausgewählt unter Hautzuständen, Krebs, Viruserkrankungen, retroviralen Erkrankungen, bakteriellen Erkrankungen, Autoimmunerkrankungen und Pilzerkrankungen.

**12.** Produkt, welches folgendes umfaßt

(a) ein aminosubstituiertes Hypocrellin, wobei das aminosubstituierte Hypocrellin ein Sonosensibilisator und/ oder ein Photosensibilisator ist und, wenn es aktiviert wird, das Potential eines immuntherapeutischen Mittels modulieren kann und das aminosubstituierte Hypocrellin aus den Verbindungen der folgenden Formeln aus-

gewählt ist

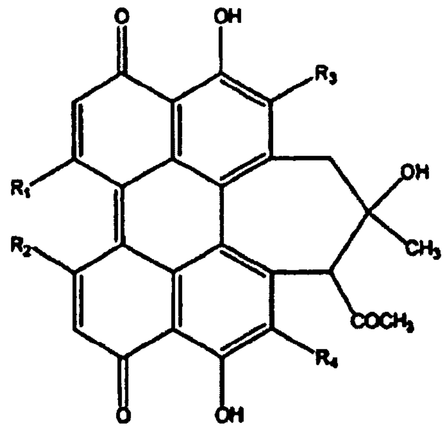

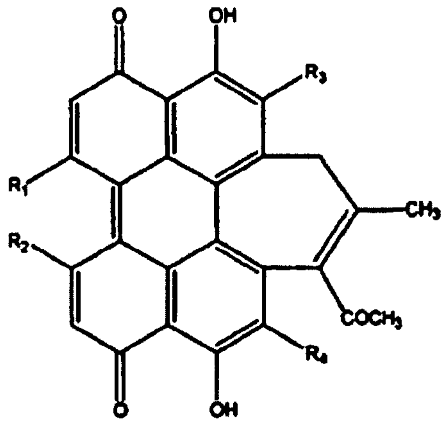

wobei

(i) $R_1$, $R_2$, $R_3$, $R_4$ $OCH_3$ oder $NHCH_2Ar$ (Ar ist eine Phenyl- oder Pyridylgruppe), $NHCH(CH_2)$, (wobei -CH $(CH_2)_n$ eine alizyklische Gruppe ist und n = 3, 4, 5, 6 ist) sind oder
(ii) $R_1$, $R_2$, $R_3$, $R_4$ $OCH_3$ oder $NHCH_2(CH_2)_nAr$ sind, wobei Ar ein Phenyl-, ein Naphthyl-, ein polyzyklischer aromatischer oder ein heterozyklischer Rest sind und n 0-12 ist,

(b) ein immuntherapeutisches Mittel

für die gleichzeitige oder aufeinanderfolgende Verwendung zum Modulieren der Aktivität eines immuntherapeutischen Mittels oder zur Behandlung eines Zustands, ausgewählt unter Hautzuständen, Krebs, Viruserkrankungen, retroviralen Erkrankungen, bakteriellen Erkrankungen, Autoimmunerkrankungen und Pilzerkrankungen.

**Revendications**

1. Utilisation d'une hypocrelline amino-substituée pour la fabrication d'un médicament destiné à moduler l'activité d'un agent immunothérapeutique, dans laquelle l'hypocrelline amino-substituée est un sonosensibilisateur et/ou un photosensibilisateur, et dans laquelle l'hypocrelline amino-substituée est capable, lorsqu'elle est activée, de moduler le potentiel d'un agent immunothérapeutique.

2. Utilisation selon la revendication 1, dans laquelle l'hypocrelline amino-substituée peut être activée en utilisant un son d'une fréquence prédéterminée.

3. Utilisation selon la revendication 1, dans laquelle l'hypocrelline amino-substituée peut être activée en utilisant une lumière d'une longueur d'onde prédéterminée.

4. Utilisation selon la revendication 1, 2 ou 3, dans laquelle un agent immunothérapeutique est choisi dans le groupe constitué par un anticorps, un antigène, une cytokine ou un immunoadjuvant.

5. Utilisation selon l'une quelconque des revendications 1 à 4, dans laquelle l'hypocrelline amino-substitué est non toxique à des concentrations élevées dans son état non activé et toxique à de faibles concentrations dans son état activé.

6. Utilisation selon l'une quelconque des revendications 1 à 5, dans laquelle l'hypocrelline amino-substituée est choisie dans le groupe constitué par l'hypocrelline B butylaminée ; la 2-(N,N-diméthylamino)-propylamine-hypocrelline B ; l'hypocrelline B éthanolaminée ; et la 1,12-bis[2-(acétyloxy)propyl]-2,4,6,7,9,11-hexaméthoxy-3,10-pérylènedione.

7. Utilisation selon l'une quelconque des revendications 1 à 5, dans laquelle l'hypocrelline amino-substituée est choisie parmi les composés de formules :

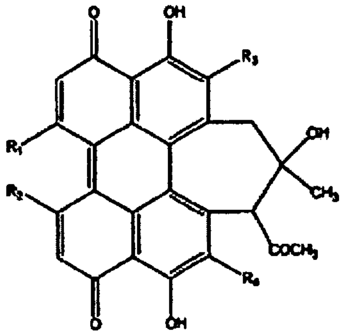

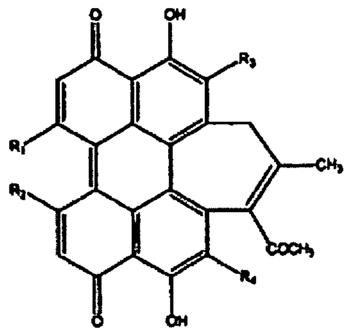

où $R_1$, $R_2$, $R_3$, $R_4$ représentent $OCH_3$ ou $NHCH_2Ar$ (Ar représente un groupe phényle ou un groupe pyridyle), NHCH $(CH_2)_n$ (où $-CH(CH_2)_n$ représente un groupe alicyclique et n = 3, 4, 5, 6).

**8.** Utilisation selon la revendication 7, dans laquelle $R_1$, $R_2$, $R_3$ représentent $OCH_3$, et $R_4$ représente $NH(CH_2)_3CH_3$.

**9.** Utilisation selon l'une quelconque des revendications 1 à 5, dans laquelle l'hypocrelline amino-substituée est choisie parmi les composés de formules :

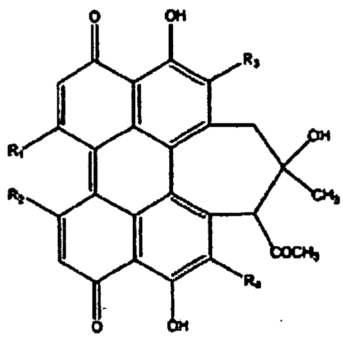

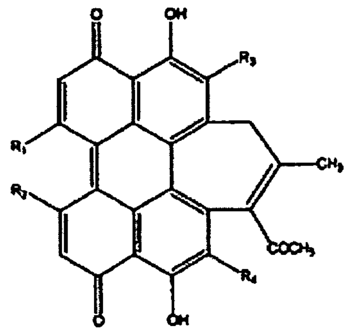

dans lesquelles $R_1$, $R_2$, $R_3$, $R_4$ représentent $OCH_3$ ou $NHCH_2(CH_2)_nAr$, où Ar représente un groupe phényle, un groupe naphtyle, un groupe aromatique polycyclique ou un groupe hétérocyclique, et n vaut 0 à 12.

**10.** Utilisation d'une composition comprenant une hypocrelline amino-substituée et un agent immunothérapeutique pour la fabrication d'un médicament destiné à moduler l'activité d'un agent immunothérapeutique, selon l'une quelconque des revendications 1 à 9.

**11.** Utilisation selon l'une quelconque des revendications 1 à 9, pour la fabrication d'un médicament destiné à traiter un état choisi parmi des maladies de la peau, un cancer, des maladies virales, des maladies rétrovirales, des maladies bactériennes, des maladies autoimmunes et des mycoses.

**12.** Produit comprenant

(a) une hypocrelline amino-substituée, ladite hypocrelline amino-substituée étant un sonosensibilisateur et/ou un photosensibilisateur et étant capable lorsqu'elle est activée de moduler le potentiel d'un agent immunothérapeutique, et l'hypocrelline amino-substituée étant choisie parmi les composés de formules :

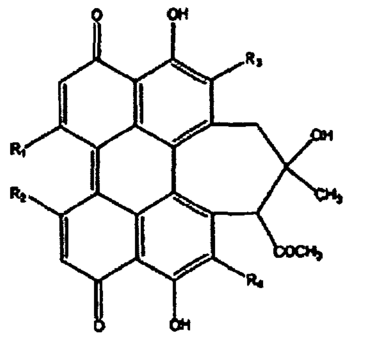

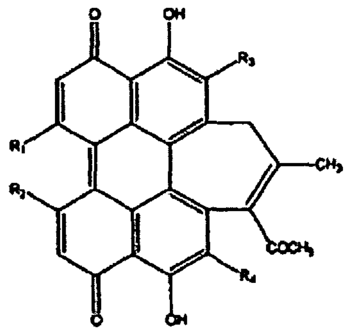

dans lesquelles

(i) $R_1$, $R_2$, $R_3$, $R_4$ représentent $OCH_3$ ou $NHCH_2Ar$ (Ar représente un groupe phényle ou un groupe pyridyle), $NHCH(CH_2)_n$ (où $-CH(CH_2)_n$ représente un groupe alicyclique et n = 3, 4, 5, 6) ; ou
(ii) $R_1$, $R_2$, $R_3$, $R_4$ représentent $OCH_3$ ou $NHCH_2(CH_2)_nAr$, où Ar représente un groupe phényle, un groupe naphtyle, un groupe aromatique polycyclique ou un groupe hétérocyclique, et n vaut 0 à 12 ;

(b) un agent immunothérapeutique,

pour une utilisation simultanée ou successive dans la modulation de l'activité d'un agent immunothérapeutique ou le traitement d'un état choisi parmi des maladies de la peau, un cancer, des maladies virales, des maladies rétrovirales, des maladies bactériennes, des maladies autoimmunes et des mycoses.

# Figure 1

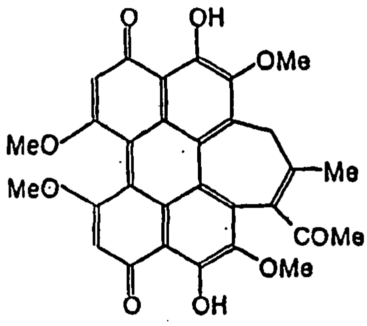

a. HYPOCRELLIN B (HB)

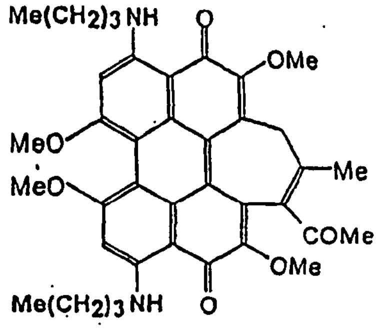

b. HBBA-R2

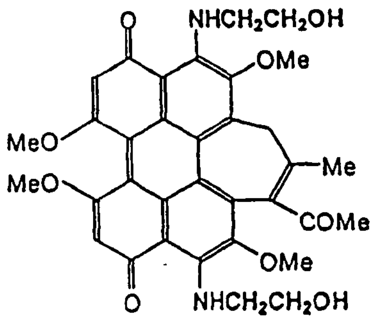

c. HBEA-R1

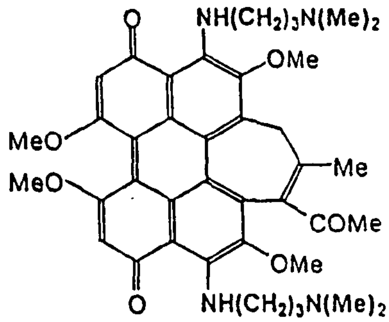

d. HBDP-R1

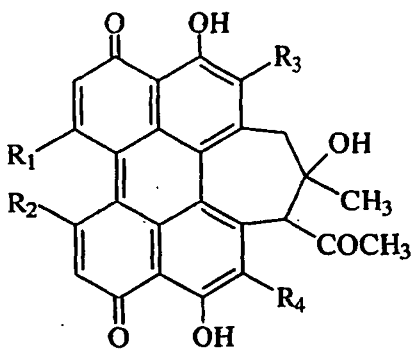

V

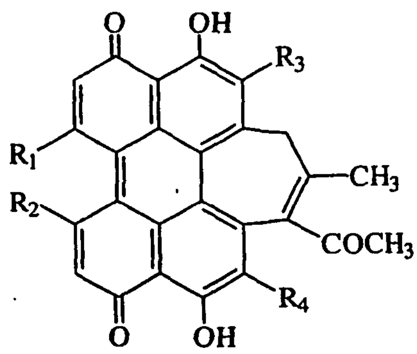

VI

# Figure 2

Fig. 3A

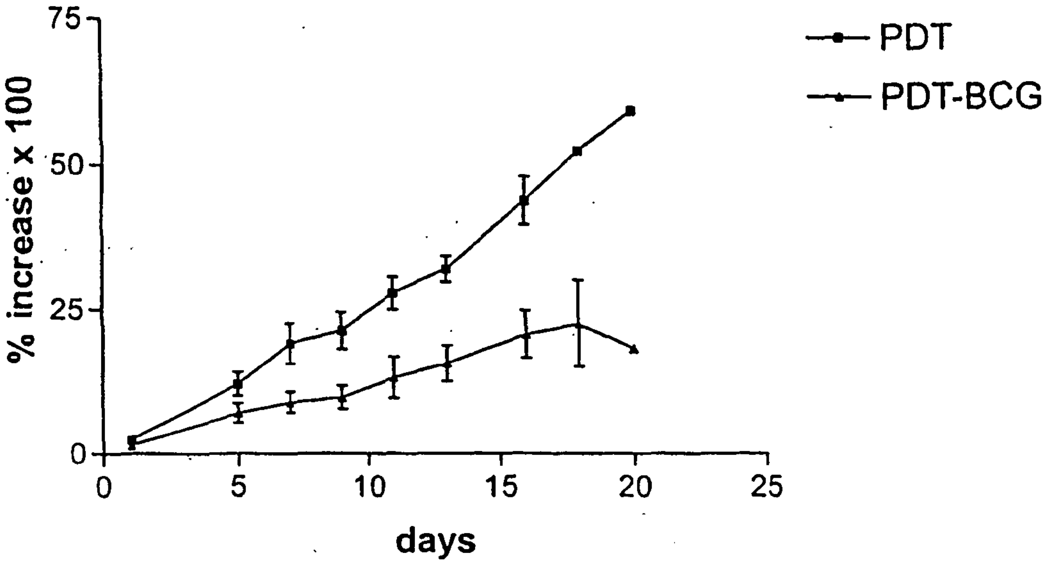

% increase x 100
75
50
25
0
0
5
10
15
20
25
days
PDT
PDT-BCG


Fig. 3B

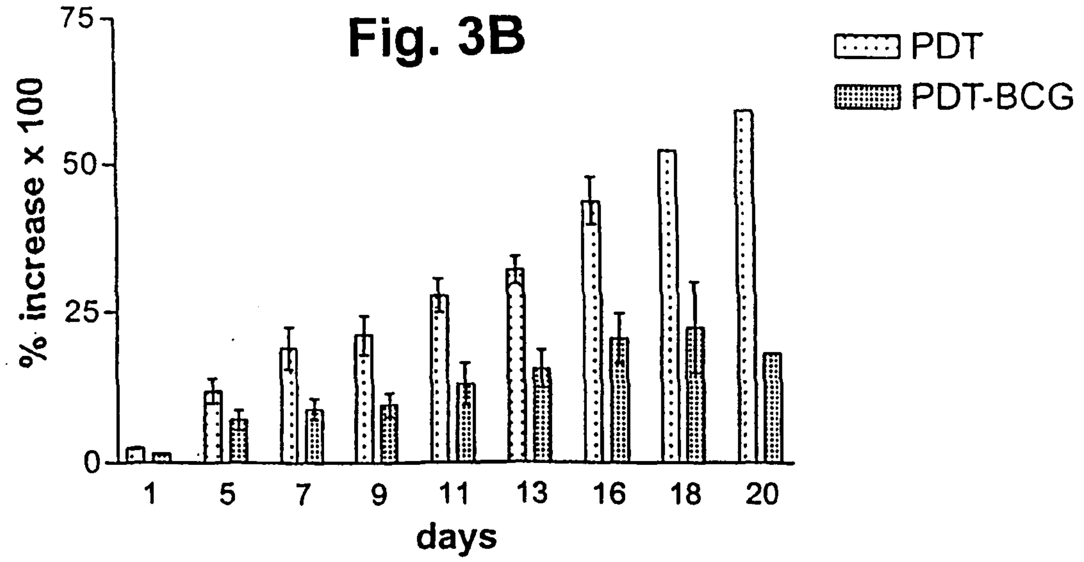

% increase x 100
75
50
25
0
1
5
7
9
11
13
16
18
20
days
PDT
PDT-BCG

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4722906 A
- US 3959078 A


### Non-patent literature cited in the description


- **DIWU et al.** *J. Photochem. Photobiol. A: Chem.,* 1992, vol. 64, 273
- Hypocrellin A, a new drug for photochemotherapy. **ZHANG ; WAN et al.** Kexue Tongbao. 1981, vol. 26, 1040
- **WEISS et al.** *Prog. Chem. Org. Nat. Prod.,* 1987, vol. 52, 1
- **DIWU et al.** *Photochem & Photobiol.,* 1990, vol. 52, 609-616
- **DIWU et al.** *Pharmac. Ther.,* 1994, vol. 63, 1
- **LIU et al.** Synthetic studies in novel hypocrellin B derivatives. *Tetrahedron,* 1993, vol. 49, 10785
- **DIWU et al.** *Anti-Cancer Drug Design,* 1993, vol. 8, 129-143
- **DIWU et al.** *Biochem. Pharmacol.,* 1994, vol. 47, 373-389
- **LISE LD ; AUDIBERT F.** Immunoadjuvants and analogs of immunomodulatory bacterial structures. *Curr Opin Immunol,* 1989, vol. 2, 269-274
- **GOFF et al.** *British Journal of Cancer,* 1996, vol. 74, 1194-1198
- Remington's Pharmaceutical Science. Mack Publishing Co, 1982
- **HUNT DW ; CHAN AH.** *Expert Opinion on Investigational Drugs,* 2000, vol. 9, 807
- **KORBELIK M.** *Journal of Clinical Laser Medicine & Surgery,* 1996, vol. 14, 329
- **KORBELIK M ; DOUGHERTY GJ.** *Cancer Research,* 1999, vol. 59, 1941
- **ABDEL-HADY ES ; MARTIN-HIRSCH P ; DUGGAN-KEEN M ; STERN PL ; MOORE JV ; CORBITT G ; KITCHENER HC ; HAMPSON IN.** *Cancer Research,* 2001, vol. 61, 192
- **DOUGHERTY TJ ; GOMER CJ ; HENDERSON BW ; JORI G ; KESSEL D ; KORBELIK M ; MOAN J ; PENG Q.** *Journal of the National Cancer Institute,* 1998, vol. 90, 889
- **KORBELIK M ; KROSL G ; KROSL J ; DOUGHERTY GJ.** *Cancer Research,* 1996, vol. 56, 5647
- **OCHSNER M.** *Journal of Photochemistry & Photobiology. B - Biology,* 1997, vol. 39, 1
- **DE VREE WJ ; ESSERS MC ; KOSTER JF ; SLUITER W.** *Cancer Research,* 1997, vol. 57, 2555
- **GOLLNICK SO ; LIU X ; OWCZARCZAK B ; MUSSER DA ; HENDERSON BW.** *Cancer Research,* 1997, vol. 57, 3904
- **KROSL G ; KORBELIK M ; DOUGHERTY GJ.** *British Journal of Cancer,* 1995, vol. 71, 549
- **KORBELIK M ; KROSL G.** *Photochemistry & Photobiology,* 1994, vol. 60, 497
- **HENDRZAK-HENION JA ; KNISELY TL ; CINCOTTA L ; CINCOTTA E ; CINCOTTA AH.** *Photochemistry & Photobiology,* 1999, vol. 69, 575
- **DE VREE WJ ; ESSERS MC ; DE BRUIJN HS ; STAR WM ; KOSTER JF ; SLUITER W.** *Cancer Research,* 1996, vol. 56, 2908
- **KORBELIK M ; CECIC I.** *Cancer Letters,* 1999, vol. 137, 91
- **KORBELIK M ; SUN J.** *International Journal of Cancer,* 2001, vol. 93, 269
- **BELLNIER DA.** *Journal of Photochemistry & Photobiology. B - Biology,* 1991, vol. 8, 203
- **KROSL G ; KORBELIK M ; KROSL J ; DOUGHERTY GJ.** *Cancer Research,* 1996, vol. 56, 3281
- **CHO YH ; STRAIGHT RC ; SMITH JA.** *Journal of Urology,* 1992, vol. 147, 743
- **KORBELIK M ; SUN J ; POSAKONY JJ.** *Photochemistry & Photobiology,* 2001, vol. 73, 403
- **KORBELIK M ; CECIC I.** *Journal of Photochemistry & Photobiology. B - Biology,* 1998, vol. 44, 151
- **MYERS RC ; LAU BH ; KUNIHIRA DY ; TORREY RR ; WOOLLEY JL ; TOSK J.** *Urology,* 1989, vol. 33, 230
- **ESTEY EP ; BROWN K ; DIWU Z ; LIU J ; LOWN JW ; MILLER GG ; MOORE RB ; TULIP J ; MCPHEE MS.** *Cancer Chemotherapy & Pharmacology,* 1996, vol. 37, 343
- **MILLER GG ; BROWN K ; BALLANGRUD AM ; BARAJAS O ; XIAO Z ; TULIP J ; LOWN JW ; LEITHOFF JM ; ALLALUNIS-TURNER MJ ; MEHTA RD.** *Photochemistry & Photobiology,* 1997, vol. 65, 714